# EUROPEAN PATENT SPECIFICATION

(11) **EP 3 047 828 B1**
(45) Date of publication and mention of the grant of the patent: **14.03.2018**
(21) Application number: 14822719.2
(22) Date of filing: 14.01.2014
(51) Int. Cl.: A61F 13/15

(54) **MALE ABSORBENT PAD**
SAUGPOLSTER FÜR MÄNNER
GARNITURE ABSORBANTE POUR HOMME

(30) Priority: 20.09.2013 JP 2013196227
(43) Date of publication of application: 27.07.2016
(73) Proprietor: Unicharm Corporation, Ehime 799-0111 (JP)
(72) Inventor: FUJIMOTO, Kazuya, Kanonji-shi Kagawa 769-1602 (JP); NAKAJIMA, Kaiyo, Kanonji-shi Kagawa 769-1602 (JP)
(74) Representative: Peter, Julian
(86) International application number: PCT/JP2014/050476
(87) International publication number: WO 2015/004929

(56) References cited:
- WO-A1-2011/162657
- JP-A- H09 206 324
- JP-A- H09 299 392
- JP-A- S61 217 160
- JP-A- 2001 129 012
- JP-A- 2003 265 519
- JP-A- 2006 212 452
- JP-A- 2009 207 516
- JP-A- 2009 207 516
- JP-A- 2009 516 539
- US-B2- 8 277 426

## Description

### {Technical Field}

The present invention relates to male absorbent pads each having a plurality of concave grooves.

### {Background}

Liquid-absorbent pads adapted to be used together with underwear such as pants are conventionally well known. For example, an absorbent pad disclosed in the Patent Literature 1 includes a topsheet, a backsheet and a liquid-absorbent core interposed between the topsheet and the backsheet. The liquid-absorbent core is formed with a pair of longitudinal linear grooves extending in a longitudinal direction symmetrically about a longitudinal axis and with a pair of transverse linear grooves extending in the transverse direction symmetrically about a transverse axis.

### {Citation List}

### {Patent Literature}

{PTL 1}: JP 2009 - 82480 A
Further prior art in this technical field is disclosed in documents JP 2009 207516 A and WO2011/162657 A1.

### {Summary}

### {Technical Problem}

With such absorbent pads put on the wearer's body, it may improve the fit of the pad to the wearer's skin because the liquid-absorbent core is bent along the longitudinal linear grooves and along the transverse linear grooves in such a manner that the end regions of the pad opposite in the longitudinal direction may be convexed toward the wearer's skin and the portion of the liquid-absorbent core overlapping the longitudinal linear grooves or defined between the longitudinal linear grooves may be convexed toward the wearer's skin side. However, when the absorbent pad of such arrangement is used as male absorbent pad, the portions of the pad convexed toward the wearer's skin may get in touch with the male external genitals, particularly penis and create a feeling of discomfort against the wearer. The portions of the pad convexed inward may also be pressed against the male external genitals, particularly the penis, to deform the liquid-absorbent core irregularly and a gap may be formed between the absorbent pad and the wearer's body. Consequently, the fit to the genital area may be deteriorated to cause a urine leakage.

An object of the present invention is to improve the prior art by partially convexing the pad outward along the wearer's body shape, thereby providing male absorbent pads assuring an improved fit.

### {Solution to Problem}

To achieve the object set forth above, the present invention is directed to a male absorbent pad according to claim 1.

### {Advantageous Effects of Invention}

According to one or more embodiments of male absorbent pads, it is possible to deform convexly the absorbent pad outward, thereby to wrap around the male external genitals thereby providing a good fit of male absorbent pads to wearers.

### {Brief Description of Drawings}

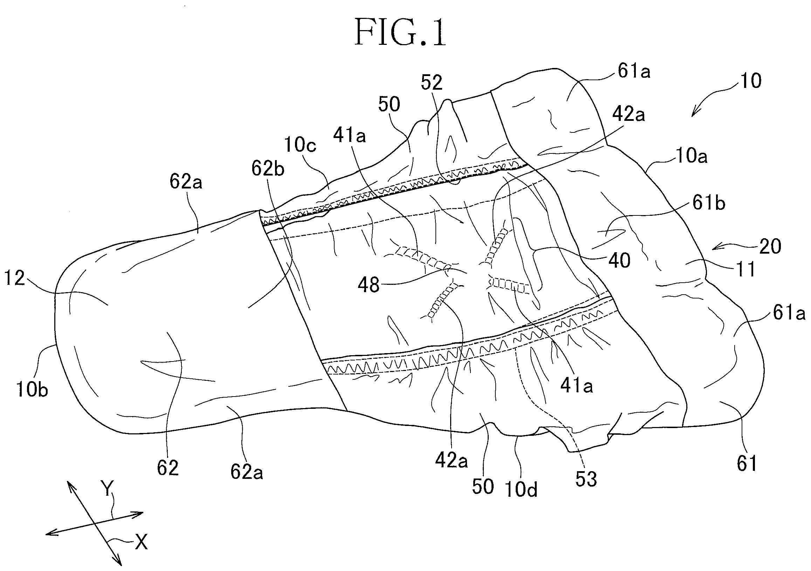

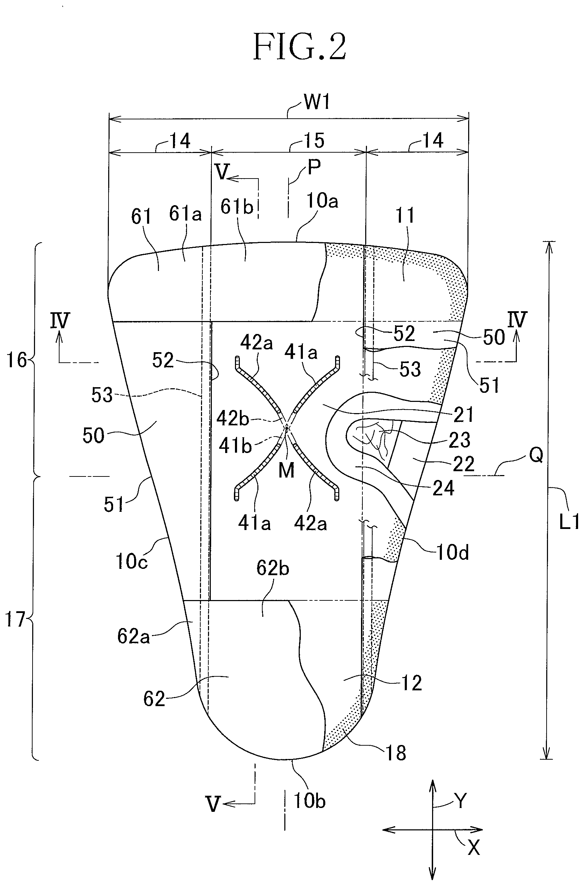

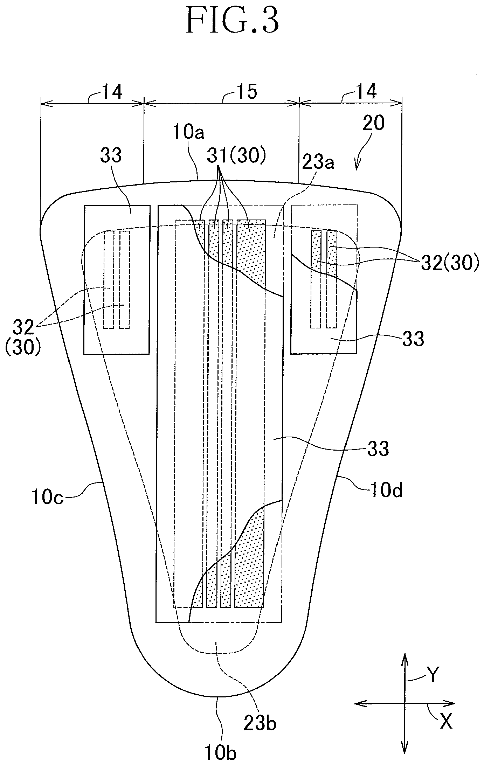

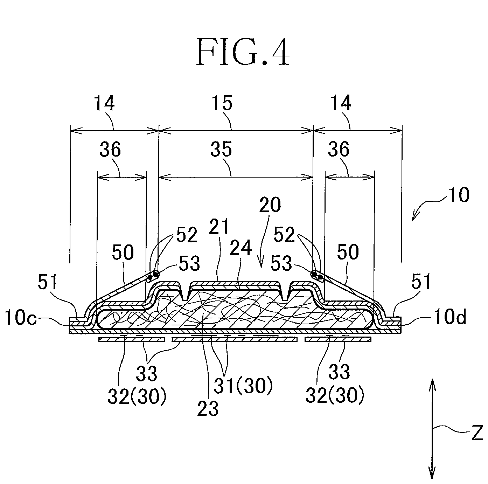

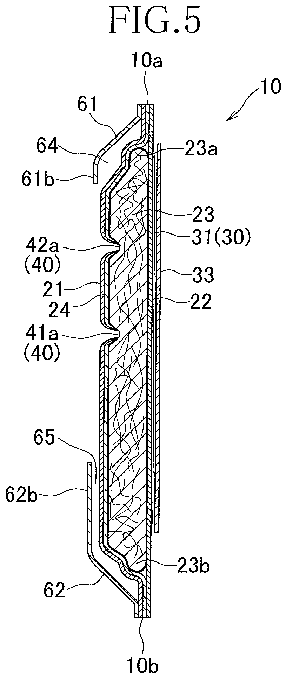

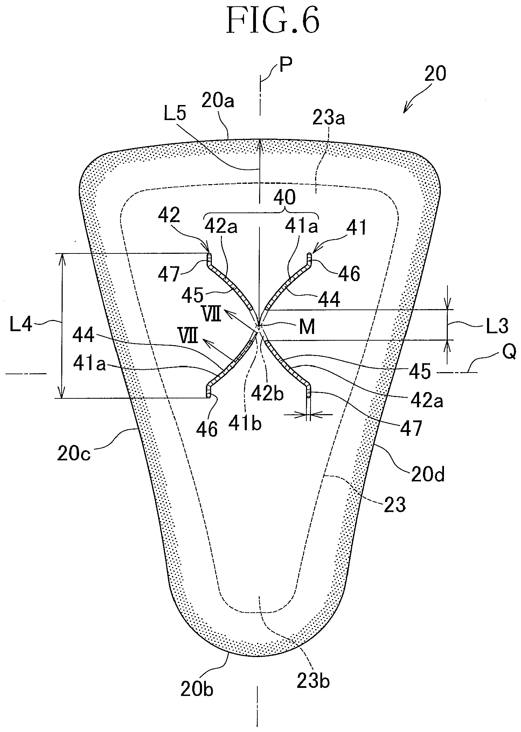

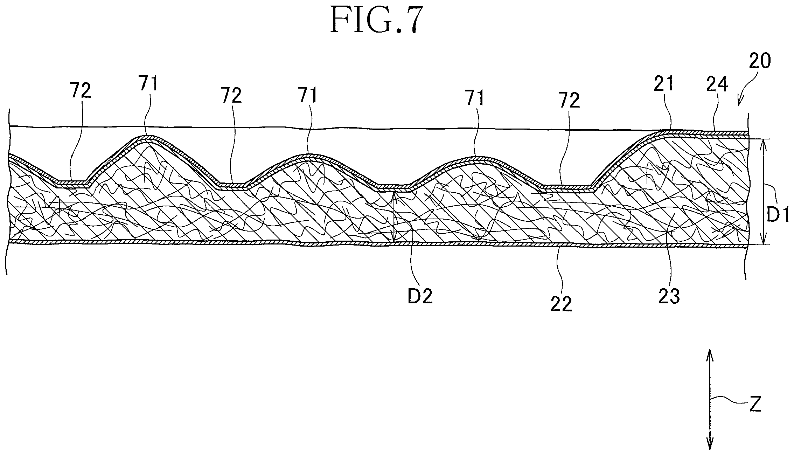

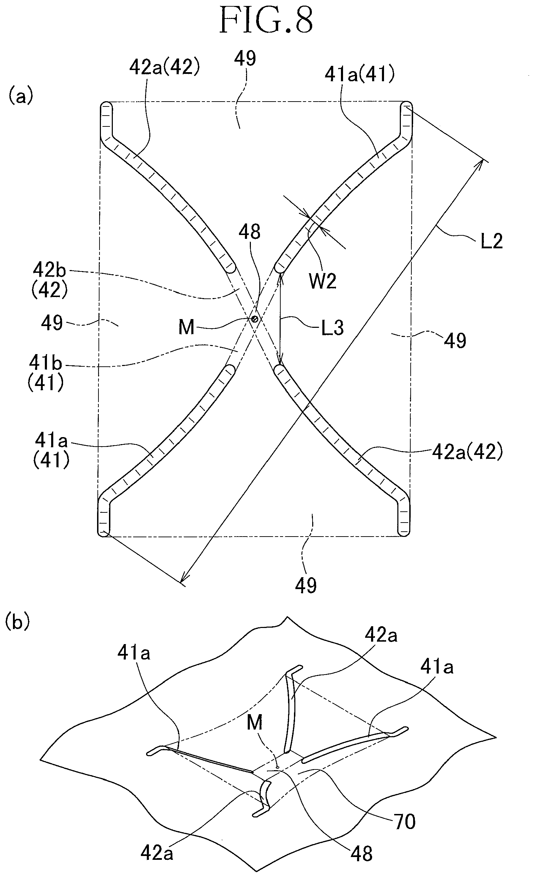

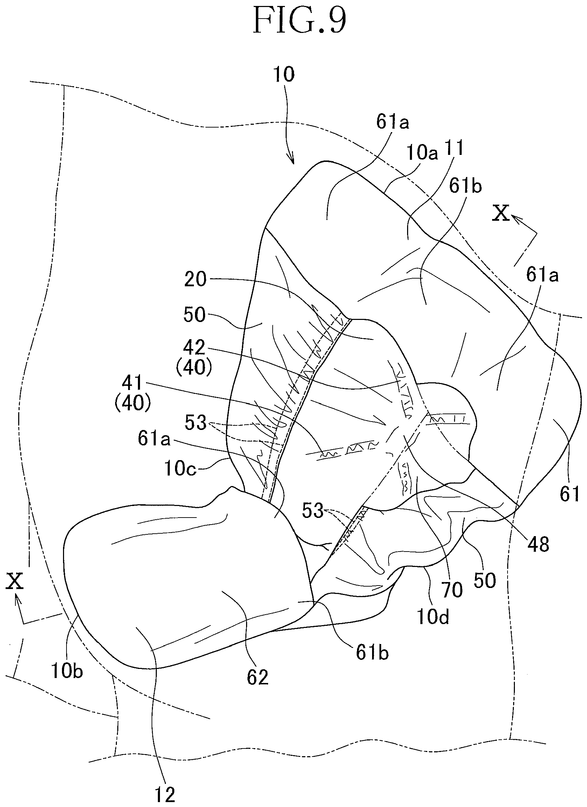

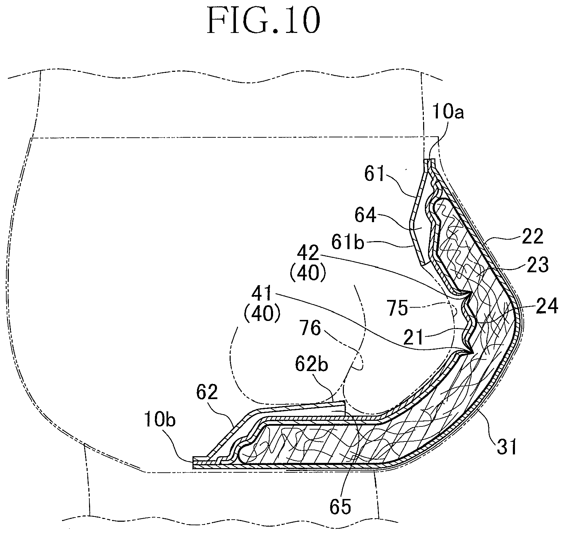

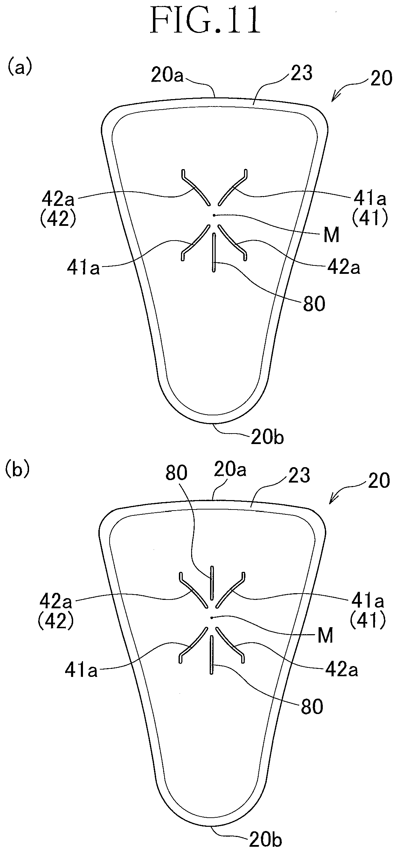

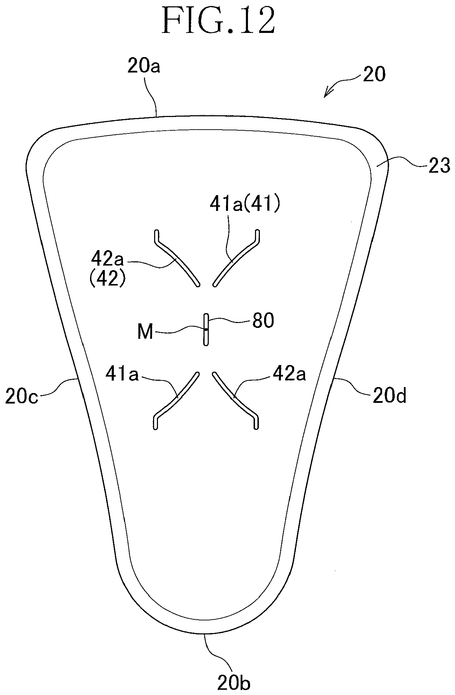

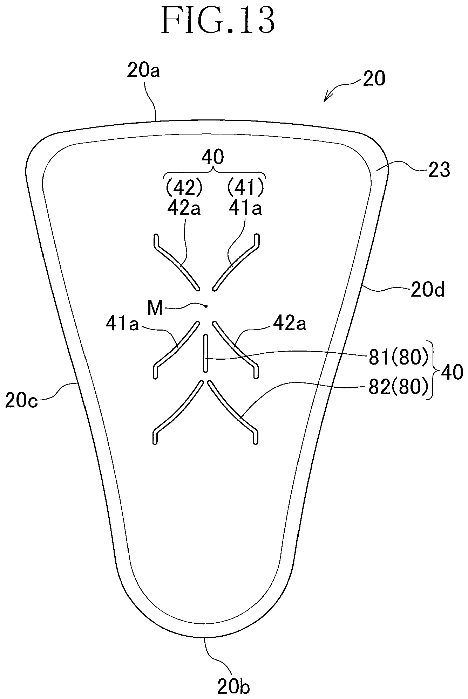

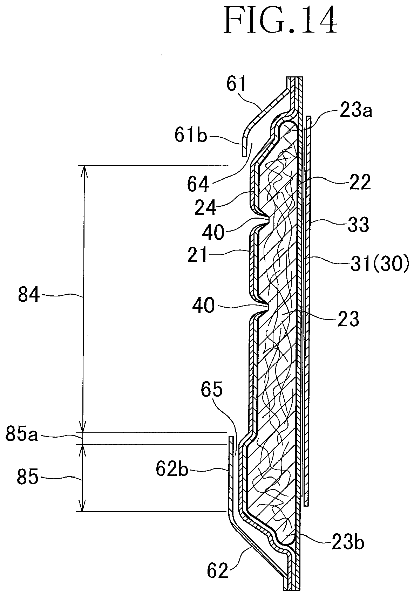

The drawings illustrate specific embodiments of the present invention including optional and preferred embodiments and essential features of the invention.
{Fig. 1} A perspective view of an absorbent pad illustrated as an example of a male absorbent pad according to the present invention.
{Fig. 2} A partially cutaway plan view of the absorbent pad.
{Fig. 3} A partially cutaway rear view of the absorbent pad.
{Fig. 4} A sectional view taken along line IV - IV in Fig. 2.
{Fig. 5} A schematic sectional view taken along line V - V in Fig. 2.
{Fig. 6} A plan view of a main body of the pad.
{Fig. 7} A sectional view taken along line VII - VII in Fig. 6.
{Fig. 8} A partially enlarged view of the main body of the pad.
{Fig. 9} A perspective view of the absorbent pad put on the wearer's body.
{Fig. 10} A schematic sectional view taken along line X - X in Fig. 9.
{Fig. 11} A plan view illustrating another embodiment of the absorbent pad.
{Fig. 12} A plan view illustrating still another embodiment of the absorbent pad.
{Fig. 13} A plan view illustrating yet another embodiment of the absorbent pad.
{Fig.14} A sectional view similar to Fig. 5, illustrating further another embodiment of the absorbent pad.

### {Description of Embodiments}

The embodiments described below is directed to the male absorbent pads illustrated in Figs. 1 through 14, including optional and preferred embodiments and essential features of the invention.

### <First Embodiment>

Figs. 1 through 5 illustrate an example of male absorbent pads 10 according to the present invention. An absorbent pad 10 has a longitudinal direction Y and a transverse direction X being orthogonal to each other, a skin facing surface and a non-skin-facing surface opposited to the former, a longitudinal axis P extending in the longitudinal direction Y so as to bisect a dimension W1 in the transverse direction X and a transverse axis Q extending in the transverse direction X so as to bisect a dimension L1 in the longitudinal direction Y. The absorbent pad 10 is formed symmetrically about the longitudinal axis P so as to be defined by curved first and second end edges 10a, 10b opposited to each other in the longitudinal direction Y and both lateral edges 10c, 10d extending in the longitudinal direction Y between the first and second end edges 10a, 10b. In addition, the absorbent pad 10 has a first end area 11 extending on the side of the first end edge 10a, a second end area 12 extending on the side of the second end edge 10b and both lateral areas 14 defined between these two end areas and extending in the longitudinal direction Y. The first end edge 10a is more gently curved than the second end edge 10b and has a dimension in the transverse direction X larger than that of the second end edge 10b so that the first end area 11 may have a width larger than that of the second end area 12. In this regard, as long as advantageous effects of the present invention as described later are ensured, the absorbent pad 10 may have various well known planar views, for example, circular or rectangular planar views other than that described above. For convenience of description, the absorbent pad 10 is sectionalized to a first zone 16 extending from the transverse axis Q to the first end edge 10a and a second zone 17 extending from the transverse axis Q to the second end edge 10b.

The absorbent pad 10 includes a pad main body 20 defining a whole shape thereof, a pair of barrier-flaps (containment sheets) 50 respectively located in both the lateral areas of the pad main body 20 and first and second cover sheets 61, 62 located in the first end area 11 and the second end area 12, respectively, to cover the absorbent pad 20. The pad main body 20, the barrier-flaps 50 and the first and second cover sheets 61, 62 are layered to each other sealing means 18 extending along an outer peripheral border of the absorbent pad 10.

The pad main body 20 includes a topsheet 21 on the side of the skin facing surface, a backsheet 22 on the side of the non-skin facing surface and a liquid-absorbent core 23 interposed between the topsheet 21 and the backsheet 22. In addition, a cushion sheet 24 may be optionally located so as to be interposed between the topsheet 21 and the liquid-absorbent core 23. The pad main body 20 is disposed with flexion-inducing grooves 40 recessed from the side of the skin-facing surface toward the side of the non-skin-facing surface.

The topsheet 21 and the cushion sheet 24 are formed of fibrous nonwoven fabrics having a liquid-permeability and, to form the topsheet 21 and the cushion sheet 24, various well-known types of fibrous nonwoven fabrics, for example, hydrophilic spunbond fibrous nonwoven fabrics, point-bond fibrous nonwoven fabrics or air-through nonwoven fabrics each having a unit mass in a range of about 15 g/m² to about 40 g/m² may be used. The backsheet 22 is formed of liquid-impermeable fibrous nonwoven fabrics, plastic films or laminate sheets formed of them. When the backsheet 22 is formed of fibrous nonwoven fabrics, for example, spunbond/meltblown/spunbond (SMS) fibrous nonwoven fabrics or spunbond fibrous nonwoven fabrics each having a mass per unit area in a range of about 10 g/m² to about 30 g/m² may be used.

If a mass per unit area of the topsheet 21 and the cushion sheet 24 is less than about 15 g/m², strength thereof is comparatively low and when the flexion-inducing grooves 40 are provided, these sheets 21, 24 may be partially broken and the liquid-absorbent core 23 may exposed. When a mass per unit area of these sheets 21, 24 exceeds about 40 g/m², an assembly of these sheets 21, 24 becomes comparatively bulky and makes it difficult to form the liquid-absorbent core 23 together with these layered sheets with the flexion-inducing grooves 40. The cushion sheet 24 is more bulky than the topsheet 21 to improve its cushion effect. In a central area of the cushion sheet 24 defined between the pair of the barrier flaps 50, within which the topsheet 21 is exposed, may be disposed with a colored element. Such a colored element located in the central area of the cushion sheet 24 help the wearer put the male external genitals properly in contact with the interior surface of the pad main body 20.

The absorbent core 23 has nearly the same contour as the absorbent pad 10 and includes absorbent materials formed from a mixture of wood fluff pulp, superabsorbent polymer particles (SAP) and optionally thermoplastic fibers, and liquid-diffusive sheets such as tissue paper to wrap the absorbent materials.

Each of the barrier-flaps 50 is formed of liquid-impermeable fibrous nonwoven fabrics having a shape gradually tapered from the first end edge 10a toward the second end edge 10b. The barrier-flap 50 has a proximal edge portion 51 fixed together with the other sheet members by the thermal fusion bonding means 18, both end portions fixed to the topsheet 21 in the first and second end areas 11, 12 with hot melt adhesive and a sleeve distal edge portion 52 extending in the longitudinal direction Y between the both fixed end portions and formed by folding back the inner side edge of the barrier-flap 50 inward. To the distal edge portion 52 of the barrier-flap 50, linear flap elastic element 53 such as strings or strands is contractibly attached. In the absorbent pad 10 put on the wearer's body, the distal edge portion 52 spaces away from the topsheet 21 toward the wearer's body under contraction of the flap elastic element 53 to form each of barrier flaps to prevent urine from leaking sideways.

In the pad main body 20, the topsheet 21, the cushion sheet 24 and the backsheet 22 extend outward beyond the outer peripheral edge of the liquid-absorbent core 23 and the respective portions of these sheets extending outward beyond the outer peripheral edge are bonded together by the sealing means 18 of the other well-known bonding means such as hot melt adhesives or thermal fusion bonding means. These outwardly extending portions cooperate with the respective proximal edge portions 51 to form side flaps extending outward in the transverse direction X from the both lateral edges of the liquid-absorbent core 23. These outwardly extending portions cooperate also with both the fixed end portions of the respective barrier flaps 50 and both the fixed lateral portions 61a, 62a of the first and second cover sheets 61, 62, respectively, to form end flaps defined outside the both end edges of the liquid-absorbent core 23 as viewed in the longitudinal direction Y.

Referring to Fig. 3, on a back side of the absorbent pad 10, i.e., on the exterior surface of the backsheet 22, arranged are a plurality of linear fastening zones 30 formed from pressure-sensitive adhesives and extending in the longitudinal direction Y so that the absorbent pad 10 may be fixed to clothes such as undergarment by these linear fastening zones 30. The linear fastening zones 30 includes central linear fastening zones 31 extending from the first end area 11 to the second end area 12 across the transverse axis Q and lateral linear fastening zones 32 arranged on the side of the first end area 11 symmetrically about the central linear fastening zones 31. The central linear fastening zones 31 and the lateral linear fastening zones 32 are covered with separators 33 made of plastic films or fibrous nonwoven fabrics.

Referring to Figs. 2 and 4, the liquid-absorbent core 23 has a central region 35 in which the flexion-inducing grooves 40 are arranged, and both lateral areas 36 arranged on both sides of the central area 35. The central area 35 of the liquid-absorbent core 23 has a thickness dimension larger than that of both the lateral areas 36. In the vicinity of boundaries between the central area 35 and both the lateral areas 36, the liquid-absorbent core 23 varies gradually increases in thickness and a mass per unit area of the liquid-absorbent core 23 in the central area 35 ranges 200 g/m² to 800 g/m² and a mass per unit area of the liquid-absorbent core 23 in both the lateral area 36 ranges 100 g/m² to 300 g/m². When a mass per unit area in the central area 35 is less than 200 g/m², the depth of the flexion-inducing grooves 40 may become too shallow to function as the flexion-inducing grooves 40. When a mass per unit area of the liquid-absorbent core 23 in the central area 35 exceeds 800 g/m², an excessive thickness may create a feeling of discomfort against the wearer. When a mass per unit area in both the lateral areas36 is less than 100 g/m², a stiffness may be too low to maintain a desired shape and when a mass per unit area exceeds 300 g/m², an excessive thickness may create a feeling of discomfort against the wearer.

Referring to Fig. 5, the liquid-absorbent core 23 is shaped so that a thickness thereof may be relatively thinner in both end edge portions 23a, 23b thereof. By locally adjusting the thickness of the liquid-absorbent core 23 so as to be thinner in both the lateral areas 36 than in the central area 35 and, with respect to the central area 35, locally adjusting the central area 35 so as to be thinner in both the end edge portions 23a, 23b than the remaining portion the liquid-absorbent core 23, it is possible to control an acute change in thickness (difference in level) in the outer peripheral edge of the liquid-absorbent core 23 and the end flaps and the side flaps adjoining to thereto. In this way, an outer contour of the liquid-absorbent core 23 should not convexly appear on the outer surface of the undergarment to which the absorbent pad 10 is attached, hence, it is possible to adapt the outer peripheral edge portion of the absorbent pad 10 to the wearer's body.

Referring to Figs. 2 and 5, the first and second cover sheets 61, 62 respectively have both the lateral portions 61a, 62a fixed to the barrier flaps 50 on the sides of the first end edge 10a and the second end edge 10b and covering portions 61b, 62b adapted, between the fixed lateral portions 61a, 62a, to cover a central area 15 of the pad main body 20. As illustrated in Figs. 4 and 5, rising of the barrier flaps causes the respective covering portions 61b, 62b of the first and second cover sheets 61, 62 to space away from the topsheet 21 lying in the central area 15 of the pad main body 20 and, whereby first and second pockets 64, 65 are formed between the covering portions 61b, 62b and the topsheet 21.

Referring to Fig. 6, the flexion-inducing grooves 40 formed on the liquid-absorbent core 23 are defined by recess lines compressed from the topsheet 21 toward the liquid-absorbent core 23 and include a first groove 41 obliquely extending across the longitudinal axis P and a second groove 42 extending across the first groove 41. In the production process, the pad main body 20 may be subjected to the press process with use of a forming die wherein the pad main body 20 is locally compressed and heated or the pad main body 20 may be locally compressed with use of a gear having concavities and convexities arranged alternately along a circumferential surface thereof. The first and second grooves 41, 42 may be formed by partially compressing the absorbent pad 10 to ensure that a density of the absorbent core 23 along the first and second grooves 41, 42 is higher than that of the non-grooved region.

The method of forming the first and second grooves 41, 42 is not limited to the method based on the press working as in the present embodiment and it is also possible to control a mass of the liquid-absorbent core 23 in the regions corresponding to the first and second grooves 41, 42 to be lower than that in the non-grooved region. As a specific example, a forming die having an air feed bottom formed with air blow convexities appropriately protruding in the depth direction of the forming die may be used for this purpose. In this instance, a deposition thickness of the material in these air blow protrusions is thinner than that in the non-grooved region of the liquid-absorbent core 23 and a mass in the air blow protrusions is correspondingly reduced. In this manner, it is also possible to form the recessing lines by partially removing the liquid-absorbent core 23 (or partially presetting the mass to a relatively low level). Also when this forming method is adopted, for the purpose of preventing body exudates from leaking, it is preferred that the first and second grooves 41, 42 are not through-holes passing through the entire thickness of the liquid-absorbent core 23 but based grooves.

Referring to Figs. 6 through 8, the pad main body 20 has first and second end edges 20a, 20b overlapping with the first and second end edges 10a, 10b (see Fig. 2) of the absorbent pad 10 and both lateral edges 20c, 20d overlapping with both the lateral edges 20c, 20d. The first and second grooves 41, 42 obliquely extend with respect to the longitudinal axis P and intersect with each other at an intersection point M located on the longitudinal axis P in the first zone 16 (see Fig. 2). The first and second grooves 41, 42 respectively have a pair of groove segments (first segments) 41a, 42a each having protrusions 71 and depressions 72 and crossover segments (second segments) 41b, 42b defined between the groove segments 41a, 42a and including the intersection point M. According to the present embodiment, none of the groove segments 41a, 42a are not formed and indicated by imaginary lines in Fig. 6. In this regard, as long as advantageous effects of the present invention as described later are ensured, it is possible to form actual grooves also in the crossover segments 41b, 42b. The term used herein "the first and second grooves 41, 42 intersect with each other" includes the instance in which the first and second grooves 41, 42 intersect with each other in the imaginary crossover segments 41b, 42b and the instance in which the grooves are really formed also in the crossover segments 41b, 42b and these grooves really intersect with each other. However, considering that a stiffness of the pad main body 20 in the area formed with the grooves is necessarily increased, it is preferable to form none of the grooves in crossover segments 41b, 42b in which the first and second grooves 41, 42 come close to each other, thereby preventing the stiffness in the crossover segments 41b, 42b from increasing and to improve a wear comfort.

The respective groove segments 41a, 42a of the first and second grooves 41, 42 have curved segments 44, 45 extending outward from the longitudinal axis P and linear segments 46, 47 bent at respective outer ends of the curved segments 44, 45 and extending outward in the longitudinal direction Y. Dimensions of the respective segments are exemplified below. A length dimension L2 of the first and second grooves 41, 42 measured from respective end edges on the side of the first end edge 20a to respective end edges on the side of the second end edge 20b is about 90 mm, a width dimension W2 of the respective groove segments 41a, 42a is about 2 mm, a dimension L3 in the longitudinal direction Y of a region in which the crossover segments 41b, 42b are present, i.e., a non-compressed region 48 enclosed by the respective inner ends of the grooves 41a, 42a and none of the grooves are formed, is about 15 mm and a dimension L4 in the longitudinal direction Y of the respective grooves 41, 42 is about 75 mm wherein the dimension L3 ranges about 10 % to about 50 % of the dimension L4. In order that the pad main body 20 may appropriately flexed along the flexion-inducing grooves 40 to wrap around the wearer's penis, it is required that a pericentral portion of the flexion-inducing grooves 40 is located so as to be opposed to the penis. To meet such positional requirement for the flexion-inducing grooves 40, a dimension L5 in the longitudinal axis P of the pad main body 20 from the first end edge 20a to the intersection point M is preferably in a range from about 25 % to about 50 % of a total dimension in the longitudinal direction Y of the pad main body 20 (equal to the dimension L1 in the longitudinal direction Y of the absorbent pad).

The first and second grooves 41, 42 are generally curved except the linear segments 46, 47, wherein the groove segments 41a, 42a extending toward the side of the first end edge 20a and the groove segments 41a, 42a toward the side of second end edge 20b are curved in the directions different from each other. The respective groove segments 41a, 42a are arranged in a radial fashion around the intersection point M. Length dimensions of the respective groove segments 41a, 42a of the first and second grooves 41, 42 are approximately the same and the groove segments 41a, 42a opposed to each other about the longitudinal axis P are symmetrically arranged about the longitudinal axis P. The first and second grooves 41, 42 may be generally linear or may be generally curved from the inner ends to the outer ends thereof.

Referring to Fig. 8 (a) and Fig. 8 (b), in these flexion-inducing grooves 40 of such figuration, when the respective groove segments 41a, 42a of the first and second grooves 41,42 are exerted with a force directed from the outer side toward the inner side in the transverse direction X, the absorbent pad 10 is bent along these groove segments 41a, 42a and a region (convexly deformed area) surrounded by the first and second grooves 41, 42 is recessed from the side of the skin-facing surface toward the side of the non-skin-facing surface. More specifically, the groove segments 41a, 42a extending radially around the intersection point M are flexed and portions 49 defined between these segments serve as peripheral walls 70 to surround the non-compressed area48 defined around the intersection point M. In this way, the convexly deformed area is formed of a cup. To ensure that such convexly deformed area is formed, preferably four or more groove segments 41a, 42a are arranged circumferentially in the non-compressed region 48 (bottom portion) at desired intervals. The more the number of the groove segments 41a, 42a is, the higher strength of the circumferential wall 70 and the cup-shape is stably formed. However, a stiffness of the convexly deformed region may increase and the wearer may feel discomfort when this region comes in contact with the wearer's skin. To avoid such problem, the number of the groove segments 41a, 42a defining the circumferential wall 70 are preferably 4 to 8.

Referring to Fig. 7, the groove segment 41a (same in arrangement of the groove segment 42a) has the protrusions 71 and the depressions 72 alternating in the longitudinal direction thereof. A thickness dimension D2 of the liquid-absorbent core 23 in each of the depressions 72 (corresponding to a dimension from the bottom surface to the position of the depressions 72) is about 10 % to about 50% of a thickness dimension D1 of the portion of the liquid-absorbent core 23 in which none of the flexion-inducing grooves 40. When the thickness dimension D2 is less than 10% of the thickness dimension D1, the groove segment 41a is relatively deep and consequently a stiffness of the liquid-absorbent core 23 in the compressed portions becomes relatively deep and the wearer may feel discomfort. Meanwhile, when the thickness dimension D2 is 50 % or more of the thickness dimension D1, the grooves become relatively shallow and a differential stiffness relative to the other region becomes small. Consequently the liquid-absorbent core 23 may not be deformed to the desired shape. The groove segments 41a, 42a are embossed/debossed to arrange the protrusions 71 and the depressions 72 alternately in the longitudinal direction and, in consequence, these groove segments 41a, 42a are not easily flexed in the width direction in comparison with the flat groove having none of the depressions and the protrusions. Consequently, the groove segments 41a, 42a defining the peripheral wall 70 should not get out of shape when the absorbent pad 10 is attached to the undergarment and curved.

Referring to Figs. 9 and 10, the absorbent pad 10 is curved along the wearer's body and, specifically, convexly deformed outward (toward the side of the undergarment) in the convexly deformable area along the flexion-inducing grooves 40. Under the effect of such deformation, a space defined by the circumferential wall 70 and a bottom wall (non-compressed region 48) adapted to wrap around a penis 75. If a portion of the absorbent pad 10 facing the penis 75 is planar shape, the penis 75 may be put in contact with the absorbent pad 10, a wearing feeling may deteriorated and urine leakage may occur through a gap between the absorbent pad 10 and the penis 75. For the absorbent pad 10 according to the present invention, the penis 75 is wrapped around within the space formed by the convexly deformed region, with the absorbent pad 10 put in contact with the penis 75. Hence, a wearing feeling is good and a gap which may cause urine sideways should not be formed between the penis 75 and the absorbent pad 10.

As discussed above, even in the thinnest groove 72, the respective segments 41a, 42a have the thickness dimension D2 corresponding to at least 10 % of the thickness dimension D1 and, in other words, the liquid-absorbent core 23 having a relatively high density is arranged in the groove segments 41a, 42a. Consequently, an amount of urine flowing onto these segments is rapidly absorbed therein and a part of the urine is diffused in the entire liquid-absorbent core 23 downward from above (from the first end edge 10a toward the second end edge 10b). In addition, the absorbent pad 10 is provided in a generally curved state and the barrier flaps rise under contraction of the flap elastic elements 53 to prevent effectively urine from leaking sideways and the convexly deformable region cooperates with the barrier flaps to define the space in which the penis 75 and the scrotum are wrapped around. In this way, the fit is further improved. The generally curved shape of the absorbent pad 10 is advantageous also to ensure that the barrier flaps rise under extension of the flap elastic elements 53 to prevent the urine from leaking sideways further effectively and that the penis 75 and part of the scrotum 76 are wrapped around in the space formed by the convexly deformable region in cooperation with the barrier flaps. In this way, the fit of the article is further improved.

The first and second pockets 64, 65 are formed between the first and second cover sheets 61, 62 and the topsheet 21 when the barrier flaps rise. Upon formation of the first pocket 64, if a tip of the penis 75 pointing upward (pointing toward the first end edge 10a) lies within the convexly deformed area, a urethral orifice faces the first end area 11 curved along the wearer's body shape. In such a state, the flow of urine discharged from the urethral orifice strikes the interior surface of the first pocket 64 and this first pocket 64 functions as a (upper) leakage-barrier wall to prevent the urine from being discharged so as to miss the absorbent pad 10. Upon formation of the second cover sheet 62 by the second pocket 65 and, if the tip of the penis 75 pointing downward (pointing toward the second end edge 10b) lies within the convexly deformed region, the urethral orifice faces the second end area 12 curved along a crotch region of the wearer. In such a state, the flow of urine discharged from the urethral orifice strikes the interior surface of the second pocket 65 and this second pocket 65 functions as a (lower) leakage-barrier wall to prevent the urine from leaking outward and functions also for temporary storage and absorption of the urine having flown thereinto.

### <Second Embodiment>

Referring to Fig. 11 (a), according to the present embodiment, the flexion-inducing grooves 40 have, in addition to the first and second grooves 41, 42, a third groove 80 linearly extending on the longitudinal axis P. The third groove 80 extends from the vicinity of the inner ends of the groove segments 41a, 42a in the first and second grooves 41, 42 toward the second end edge 20b and have a width dimension and a thickness dimension which are substantially the same as those of the groove segments 41a, 42a. The respective groove segments 41a, 42a, 80 radially extend around the intersection point M also in such embodiment so that the convexly deformable region may have a cup-like shape adapted to wrap around the penis and the convexly deformable region may form the space larger than that formed only by the first and second grooves 41, 42. Besides the present embodiment, the third groove 80 may extend on the longitudinal axis P to the side of the first end edge 20a or, in addition to the linearly extending on the longitudinal axis P, linear or curved grooves may be arranged on both sides of the linear groove so as to extend obliquely. The third groove 80 extending from the intersection point M to the side of the first end edge 20a makes it possible to ensure a sufficient space to wrap around the penis and to reduce a feeling of discomfort when the wearer puts on the article on him. Furthermore, the third groove 80 extending from the intersection point M to the side of the second end edge 20b makes it possible to ensure a sufficient crotch space to wrap around the penis, thereby reducing a feeling of discomfort when the wearer put the article on him.

Fig. 11 (b) illustrates one of alternatives of the second embodiment and, according to this alternative, the third grooves 80 of the flexion-inducing grooves 40 including the grooves extending on the longitudinal axis P symmetrically about the intersection point M on the side of the first end edge 20a and the second end edge 20b, respectively. The third grooves 80 radially extend together with the first and second grooves 41, 42 around the intersection point M so that the convexly deformed region may be deformed to wrap around the penis, and a space between each pair of adjacent groove segments formed on the peripheral wall 70 (see Fig. 8 (b)) may be appropriately closed to obtain a more stabilized cup-like shape.

### <Third Embodiment>

Referring to Fig. 12, the flexion-inducing grooves 40 according to the present embodiment include, in addition to the first and second grooves 41, 42, the third groove 80 extending so as to overlap the intersection point M of these first and second grooves 41, 42. The third groove 80 is distanced from the groove segments 41a, 42a of the first and second grooves 41, 42 and linearly extends on the longitudinal axis P. A width dimension and a thickness dimension of the third groove 80 are the same as those of the first and second grooves 41, 42. The third groove 80 located in the central portion of the convexly deformed region makes the exterior surface of the convexly deformed absorbent pad 10 rather tapered at a tip thereof, thereby providing a more steric convex shape. The first and second grooves 41, 42 obliquely extending outward from the longitudinal axis P in the vicinity of the both ends of the third groove 80 extending along the longitudinal axis P serve to enlarge a width dimension (a dimension in the transverse direction X) of the convexly deformed area surrounded by the flexion-inducing region, thereby ensuring a wide absorption area in the convexly deformed area. In addition, none of the grooves extending along the longitudinal axis P is formed in both the end portions in the longitudinal direction Y of the convexly deformed area and consequently it is possible to curve the convexly deformed area gently in the transverse direction X in the vicinity of both the end portions. In this way, it is possible to further reduce a feeling of discomfort when the wearer puts the article on him and to further provide a good fit to the wearer.

### <Fourth Embodiment>

Referring to Fig. 13, the flexion-inducing grooves 40 according to the present embodiment include, in addition to the first and second grooves 41, 42, third grooves 80 distanced from the first and second grooves 41, 42 toward the second end edge 20b. The third grooves 80 are arranged between the groove segments 41a, 42a and include a first groove segment 81 linearly extending on the longitudinal axis P and a pair of second groove segments 82 obliquely extending outward in the transverse direction X from the longitudinal axis P. The first and second groove segments 81, 82 have the same width dimension and the thickness dimension as those of the groove segments 41a, 42a and function to enlarge a dimension in the longitudinal direction Y of the convexly deformed area. Consequently, the absorbent pad 10 may be flexed along the first, second and third grooves 41, 42, 80 to obtain a cup-like shape larger than when the grooves are limited to the first and second grooves 41, 42.

Fig. 14 illustrates one of alternatives of the fourth embodiment, according to which the central region 35 of the liquid-absorbent core 23 has both end portions spaced apart from each other in the longitudinal direction Y and the intermediate portion including the transverse axis Q. In the central region 35 of the liquid-absorbent core 23, one end portion as viewed in the longitudinal direction Y and lying on the side of the second end area 12 has a mass per unit area larger than the remaining portions and is correspondingly thicker than the other portions. In this manner, a high-absorbent region 85 is formed. A mass per unit area of the liquid-absorbent core 23 in this high-absorbent region 85 ranges 300 g/m² to 1000 g/m². When the mass per unit area is less than 300 g/m², a sufficient absorbency may not be ensured and, when the mass per unit area exceeds 1000 g/m², the absorbent core 23 may become too bulky and create a feeling of discomfort against the wearer. A boundary 85a between the high absorbent area 85 and the other area 84 (the other end portion and the intermediate portion in the longitudinal direction Y) lies in the vicinity of an open end of the second pocket 65 formed by the second cover sheet 62. The second pocket 65 has a sufficiently high-absorptive region 85 to absorb a relatively large amount of urine staying within the second pocket 65. Furthermore, a stiffness of the absorbent pad 10 along the border 85a may be appropriately adjusted to be different from that of the other regions to flex the second pocket 65 as a whole along the wearer's crotch region, thereby providing a good fit of the absorbent pad 10. Furthermore, both the laterals of the second pocket 65 are flexed while these laterals held between the wearer's thighs so that the covering portion 62b of the second cover sheet 62 may be significantly convened to the wearer's body side, the second pocket 65 may be widely-opened and the urine flowing down on the surface of the topsheet 21 may be reliably guided into the second pocket 65. The flexion-inducing grooves 40 are formed in the intermediate portion of the central area 35 and in the other portion in the longitudinal direction Y.

Although only the pad main body 20 of the absorbent pad 10 is illustrated in Figs. 11 through 13, the arrangements other than the flexion-inducing grooves 40 formed in the pad main body 20 are similar to the arrangement of the first embodiment. So far as there is no description in the present specification, the various types of well-known materials used in the relevant technical field may be used without limitation.

The disclosure relating to the present invention described above may be arranged at least as follows.

A male absorbent pad 10 having a longitudinal direction Y and a transverse direction X being orthogonal to each other, a skin-facing surface and a non-skin-facing surface opposed to the former and a longitudinal axis P bisecting a dimension in the transverse direction X, and including a liquid-permeable topsheet 21, a liquid-impermeable backsheet 22 and a liquid-absorbent core 23 interposed between the topsheet 21 and the backsheet 22.The pad main body 20 has flexion-inducing grooves 40 recessed from the skin-facing surface toward the non-skin-facing surface, the flexion-inducing grooves 40 have a first groove 41 obliquely extending across the longitudinal axis P and a second groove 42 extending in a direction intersecting with the longitudinal axis P and with the first groove 41, and an intersection point M of the first groove 41 and the second groove 42 lies on the longitudinal axis P.

The present invention disclosed in the above paragraph {0040} may include embodiments at least as described below and these embodiments may be taken in isolation or in combination of one another.
(1) The absorbent pad 10 has first and second end areas 11, 12 spaced from and opposed to and in the longitudinal direction Y, the transverse axis Q bisecting a dimension in the longitudinal direction Y, a first zone 16 defined on the side of a first end region from the transverse axis Q and a second zone 17 defined on the side of a second end area 12 from the transverse axis Q wherein an intersection point M of the first groove 41 and the second groove 42 lies in the first zone 16.
(2) A dimension in the transverse direction X of the first end area 11 is larger than a dimension in the transverse direction X of the second end area 12.
(3) A dimension in the longitudinal direction Y along the longitudinal axis P from the end edge 10a of the first end area 11 to the intersection point M ranges about 25 % to about 50 % of a dimension L1 in the longitudinal direction Y of the absorbent pad 10 along the longitudinal axis P.
(4) The first and second grooves 41, 42 respectively have groove segments 41a, 42a radially extending from the intersection point M and crossover segments 41b, 42b having none of grooves formed therealong.
(5) The first and second grooves 41, 42 have curved shapes.
(6) The first groove 41 and the second groove 42 are arranged symmetrically located about the longitudinal axis P.
(7) The flexion-inducing grooves 40 include third groove segments 80 linearly extending on the longitudinal axis P.
(8) A third groove 80 further has a first groove segment 81 linearly extending on the longitudinal axis P and a pair of second groove segments 82 obliquely extending outward from the longitudinal axis P from the longitudinal axis P.
(9) The second groove segment 82 of the third groove 80 is curved.
(10) Each of the flexion-inducing grooves 40 is a compressed groove having protrusions and depressions arranged alternately in a length direction thereof.
(11) A mass per unit area of the liquid-absorbent core 23 is larger in a central area 35 defined between both lateral areas 36 in the transverse direction X than in these both lateral areas 36.
(12) The central area 35 of the liquid-absorbent core 23 has an intermediate portion extending about the transverse axis Q and both end portions spaced apart from each other in the longitudinal direction Y and a mass per unit area in the end portion located on the side of the second end area 12 is larger than a mass per unit area in the intermediate portion.

### {Reference Signs List}

- 10: absorbent pad
- 11: first end area
- 12: second end area
- 14: both lateral areas
- 15: central area
- 16: first zone
- 17: second zone
- 20: pad main body
- 21: topsheet
- 22: backsheet
- 23: liquid-absorbent core
- 40: flexion-inducing grooves
- 41: first groove
- 42: second groove
- 50: barrier flaps
- 61: first cover sheet
- 62: second cover sheet
- 80: third groove

## Claims

1. A male absorbent pad (10) having a longitudinal direction (Y) and a transverse direction (X) being orthogonal to each other, a skin-facing surface and a non-skin-facing surface opposed to the former and a longitudinal axis (P) bisecting a dimension in the transverse direction (X), and including a pad main body (20) having a liquid-permeable topsheet (21), a liquid-impermeable backsheet (22) and a liquid-absorbent core (23) interposed between the topsheet (21) and the backsheet (22),**characterized in that**:
the pad main body (20) has flexion-inducing grooves (40) recessed from the skin-facing surface toward the non-skin-facing surface;
the flexion-inducing grooves (40) have a first groove (41) obliquely extending across the longitudinal axis (P) and a second groove (42) extending in a direction intersecting with the longitudinal axis (P) and with the first groove (41), and imaginary crossover segments (41b, 42b) defined between the first groove (41) and the second groove (42) and including an intersection point (M) of the first groove (41) and the second groove (42) on the longitudinal axis (P) wherein none of the first groove (41) and the second groove (42) are formed in the imaginary crossover segments (41b, 42b); and
a thickness dimension and a mass per unit area of the liquid-absorbent core (23) respectively are larger in a central region (35) defined between both lateral areas (36) in the transverse direction (X) than in these both lateral regions (36).

2. The male absorbent pad (10) according to Claim 1 wherein the absorbent pad (10) has first and second end regions (11,12) spaced from and opposed to in the longitudinal direction (Y), the transverse axis (Q) bisecting a dimension in the longitudinal direction (Y), a first zone (16) defined on the side of a first end region (11) from the transverse axis (Q) and a second zone (17) defined on the side of a second end region (12) from the transverse axis (Q) wherein the intersection point (M) of the first groove (41) and the second groove (42) lies in the first zone (16).

3. The absorbent pad (10) according to Claim 1, wherein a dimension in the transverse direction (X) of the first end region (11) is larger than a dimension in the transverse direction (X) of the second end region (12).

4. The absorbent pad (10) according to Claim 2 or 3, wherein a dimension in the longitudinal direction (Y) along the longitudinal axis (P) from the end edge of the first end region (11) to the intersection point (M) is in a range of about 25 % to about 50 % of a dimension in the longitudinal direction (Y) of the absorbent pad (10) along the longitudinal axis (P).

5. The absorbent pad (10) according to any one of Claims 1 through 4, wherein the first and second grooves (41, 42) respectively have groove segments (41a, 42a) radially extending from the intersection point (M) and imaginary crossover segments (41b, 42b) having none of grooves formed therealong.

6. The absorbent pad (10) according to any one of Claim 1 through 5, wherein the first and second grooves (41, 42) have curved shapes.

7. The absorbent pad (10) according to any one of Claims 1 through 6, wherein the first groove and the second groove (41, 42) are arranged symmetrically located about the longitudinal axis (P).

8. The absorbent pad (10) according to any one of Claim 1 through 7, wherein the flexion-inducing grooves (40) include third grooves (80) linearly extending on the longitudinal axis (P).

9. The absorbent pad (10) according to Claim 8, wherein a third groove (80) further has a first groove segment (81) linearly extending on the longitudinal axis (P) and a pair of second groove segments (82) obliquely extending outward from the longitudinal axis (P).

10. The absorbent pad (10) according to Claim 9, wherein the second groove segment (82) of the third groove (80) is curved.

11. The absorbent pad (10) according to any one of Claims 1 through 10, wherein each of the flexion-inducing grooves (40) is a compressed groove having protrusions (71) and depressions (72) arranged alternately in a length direction thereof.

12. The absorbent pad (10) according to claim 1, wherein the central region of the liquid-absorbent core (23) has an intermediate portion extending about the transverse axis (X) and both end portions spaced apart from each other in the longitudinal direction (Y) and a mass per unit area in the end portion located on the side of the second end region (12) is larger than a mass per unit area in the intermediate portion.

## Patentansprüche

1. Ein absorbierendes Männerpad (10) mit einer longitudinalen Richtung (Y) und einer transversalen Richtung (X), die zueinander orthogonal sind, einer Haut-zugewandten Oberfläche und einer nicht-Haut-zugewandten Oberfläche, der vorherigen und einer longitudinalen Achse (P), die ein Ausmaß in der transversalen Richtung (X) halbiert, gegenüberliegend, und einen Padhauptkörper (20) mit einer flüssigkeitsdurchlässigen Oberlage (21), einer flüssigkeitsundurchlässigen Unterlage (22) und einem flüssigkeitsabsorbierenden Kern (23), der zwischen der Oberlage (21) und der Unterlage(22) eingeschoben ist, umfassend, **dadurch gekennzeichnet, dass**:
der Padhauptkörper (20) beugungs-induzierende Nuten (40) aufweist, die von der Haut-zugewandten Oberfläche in Richtung der nicht-Haut-zugewandten Oberfläche eingesenkt sind;
die beugungs-induzierenden Nuten (40) weisen eine erste Nut (41), die sich schräg über die longitudinale Achse (P) erstreckt, und eine zweite Nut (42), die sich in einer mit der longitudinalen Achse (P) und mit der ersten Nut (41) kreuzende Richtung erstreckt, und gedachte gekreuzte Abschnitte (41b, 42b), die zwischen der ersten Nut (41) und der zweiten Nut (42) bestimmt sind und einen Schnittpunkt (M) der ersten Nut (41) und der zweiten Nut (42) auf der longitudinalen Achse (P) umfassen, auf, wobei keine der ersten Nut (41) und der zweiten Nut (42) in den gedachten gekreuzten Abschnitten (41b, 42b) ausgebildet sind; und
eine Dickenabmessung und ein Flächengewicht des flüssigkeitsabsorbierenden Kerns (23) in einem Mittelbereich (35), der zwischen beiden lateralen Flächen (36) in der transversalen Richtung (X) bestimmt ist, jeweils größer sind als in diesen beiden lateralen Bereichen (36).

2. Das absorbierende Männerpad (10) gemäß Anspruch 1, wobei das absorbierende Pad (10) erste und zweite Endbereiche (11, 12), die von der longitudinalen Richtung (Y) beabstandet und dazu gegenüberliegend sind, eine transversale Achse (Q), die ein Ausmaß in der longitudinalen Richtung (Y) halbiert, eine erste Zone (17), die auf der Seite eines zweiten Endbereichs (12) von der transversalen Achse (Q) bestimmt ist, aufweist, wobei der Schnittpunkt (M) der ersten Nut (41) und der zweiten Nut (42) in der ersten Zone (16) liegt.

3. Das absorbierende Pad (10) gemäß Anspruch 1, wobei ein Abmaß in der transversalen Richtung (X) des ersten Endbereichs (11) größer als ein Abmaß in der transversalen Richtung (X) des zweiten Endbereichs (12) ist.

4. Das absorbierende Pad (10) gemäß Anspruch 2 oder 3, wobei ein Abmaß in der longitudinalen Richtung (Y) entlang der longitudinalen Achse (P) von der Endkante des ersten Endbereichs (11) zu dem Schnittpunkt (M) in einem Bereich von ungefähr 25 % bis ungefähr 50 % eines Abmaßes in der longitudinalen Richtung (Y) des absorbierenden Pads (10) entlang der longitudinalen Achse (P) liegt.

5. Das absorbierende Pad (10) gemäß einem der Ansprüche 1 bis 4, wobei die ersten und zweiten Nuten (41, 42) jeweils Nutabschnitte (41a, 42a), die sich von dem Schnittpunkt (M) radial erstrecken, und gedachte gekreuzte Abschnitte (41b, 42b), die keine daran entlang ausgebildeten Nuten aufweisen, aufweisen.

6. Das absorbierende Pad (10) gemäß einem der Ansprüche 1 bis 5, wobei die ersten und zweiten Nuten (41, 42) gekrümmte Formen aufweisen.

7. Das absorbierende Pad (10) gemäß einem der Ansprüche 1 bis 6, wobei die erste Nut und die zweite Nut (41, 42) symmetrisch angeordnet zur longitudinalen Achse (P) positioniert sind.

8. Das absorbierende Pad (10) gemäß einem der Ansprüche 1 bis 7, wobei die beugungs-induzierenden Nuten (40) dritte Nuten (80), die sich linear auf der longitudinalen Achse (P) erstrecken, umfassen.

9. Das absorbierende Pad (10) gemäß Anspruch 8, wobei eine dritte Nut (80) ferner einen erster Nutabschnitt (81), der sich linear auf der longitudinalen Achse (P) erstreckt, und ein Paar zweite Nutabschnitte (82), die sich schräg nach außen von der longitudinalen Achse (P) erstrecken, ausweist.

10. Das absorbierende Pad (10) gemäß Anspruch 9, wobei der zweite Nutabschnitt (82) der dritten Nut (80) gekrümmt ist.

11. Das absorbierende Pad (10) gemäß einem der Ansprüche 1 bis 10, wobei jede der beugungs-induzierenden Nuten (40) eine komprimierte Nut ist, die Vorsprünge (71) und Vertiefungen (72) aufweist, die abwechselnd in Längenrichtung dazu angeordnet sind.

12. Das absorbierende Pad (10) gemäß Anspruch 1, wobei der Mittelbereich des flüssigkeits-absorbierenden Kerns (23) einen Zwischenteil aufweist, der sich über die transversale Achse (X) und beide Endteile, die in der longitudinalen Richtung (Y) voneinander beabstandet sind, erstreckt, und ein Flächengewicht in dem Endteil, der auf der Seite des zweiten Endbereichs (12) angeordnet ist, größer als ein Flächengewicht in dem Zwischenbereich ist.

## Revendications

1. Garniture absorbante pour homme (10) présentant une direction longitudinale (Y) et une direction transversale (X) qui sont orthogonales l'une par rapport à l'autre, une surface orientée vers la peau et une surface non-orientée vers la peau en regard de la première et un axe longitudinal (P) divisant une dimension en deux dans la direction transversale (X), et comprenant un corps principal (20) de garniture ayant une feuille supérieure (21) perméable aux liquides, une feuille de support (22) imperméable aux liquides et un noyau (23) absorbant les liquides intercalé entre la feuille supérieure (21) et la feuille de support (22), **caractérisée en ce que** :
le corps principal (20) de garniture présente des rainures (40) induisant une flexion, évidées de la surface orientée vers la peau, vers la surface non-orientée vers la peau ;
les rainures (40) induisant une flexion comprennent une première rainure (41) s'étendant obliquement sur l'ensemble de l'axe longitudinal (P) et une deuxième rainure (42) s'étendant dans une direction croisant l'axe longitudinal (P) et la première rainure (41), et des segments de croisement imaginaires (41b, 42b) définis entre la première rainure (41) et la deuxième rainure (42) et comprenant un point d'intersection (M) de la première rainure (41) et de la deuxième rainure (42) sur l'axe longitudinal (P) dans laquelle ni la première rainure (41) ni la deuxième rainure (42) ne sont formées dans les segments de croisement imaginaires (41b, 42b) : et
une dimension de l'épaisseur et une masse par unité de surface du noyau (23) absorbant les liquides sont respectivement plus grandes dans une région centrale (35) définie entre les deux zones latérales (36) dans la direction transversale (X) que dans ces deux régions latérales (36).

2. Garniture absorbante pour homme (10) selon la revendication 1, dans laquelle la garniture absorbante (10) comprend des première et seconde régions d'extrémité (11,12) espacées et en regard l'une de l'autre dans la direction longitudinale (Y), l'axe transversal (Q) divisant une dimension en deux dans la direction longitudinale (Y), une première zone (16) définie sur le côté d'une première région d'extrémité (11) à partir de l'axe transversal (Q) et une seconde zone (17) définie sur le côté d'une seconde région d'extrémité (12) à partir de l'axe transversal (Q), dans laquelle le point d'intersection (M) de la première rainure (41) et de la deuxième rainure (42) se trouve dans la première zone (16).

3. Garniture absorbante (10) selon la revendication 1, dans laquelle une dimension dans la direction transversale (X) de la première région d'extrémité (11) est supérieure à une dimension dans la direction transversale (X) de la seconde région d'extrémité (12).

4. Garniture absorbante (10) selon la revendication 2 ou 3, dans laquelle une dimension dans la direction longitudinale (Y) le long de l'axe longitudinal (P), allant du bord d'extrémité de la première région d'extrémité (11) jusqu'au point d'intersection (M), est située dans une plage allant d'environ 25 % à environ 50 % d'une dimension dans la direction longitudinale (Y) de la garniture absorbante (10) le long de l'axe longitudinal (P).

5. Garniture absorbante (10) selon l'une quelconque des revendications 1 à 4, dans laquelle les première et deuxième rainures (41, 42) comprennent respectivement des segments de rainures (41a, 42a) s'étendant radialement à partir du point d'intersection (M) et des segments de croisement imaginaires (41b, 42b) sur lesquels n'est formée aucune rainure.

6. Garniture absorbante (10) selon l'une quelconque des revendications 1 à 5, dans laquelle les première et deuxième rainures (41, 42) ont des formes incurvées.

7. Garniture absorbante (10) selon l'une quelconque des revendications 1 à 6, dans laquelle les première et deuxième rainures (41, 42) sont disposées symétriquement de part et d'autre de l'axe longitudinal (P).

8. Garniture absorbante (10) selon l'une quelconque des revendications 1 à 7, dans laquelle les rainures (40) induisant une flexion comprennent des troisièmes rainures (80) s'étendant linéairement sur l'axe longitudinal (P).

9. Garniture absorbante (10) selon la revendication 8, dans laquelle une troisième rainure (80) comprend en outre un premier segment (81) de rainure s'étendant linéairement sur l'axe longitudinal (P) et une paire de seconds segments (82) de rainure s'étendant obliquement vers l'extérieur à partir de l'axe longitudinal (P).

10. Garniture absorbante (10) selon la revendication 9, dans laquelle le second segment (82) de rainure de la troisième rainure (80) est incurvé.

11. Garniture absorbante (10) selon l'une quelconque des revendications 1 à 10, dans laquelle chacune des rainures (40) induisant une flexion est une rainure comprimée comportant des saillies (71) et des enfoncements (72) disposés alternativement dans une direction de la longueur de celle-ci.

12. Garniture absorbante (10) selon la revendication 1, dans laquelle la région centrale du noyau (23) absorbant les liquides comprend une partie intermédiaire s'étendant de part et d'autre de l'axe transversal (X) et des deux parties d'extrémité espacées l'une de l'autre dans la direction longitudinale (Y) et une masse par unité de surface dans la partie d'extrémité située sur le côté de la seconde région d'extrémité (12) est supérieure à une masse par unité de surface dans la partie intermédiaire.
